# EUROPEAN PATENT APPLICATION

(11) **EP 1 046 911 A1**
(43) Date of publication of application: **25.10.2000**
(21) Application number: 99201279.9
(22) Date of filing: 23.04.1999
(51) Int. Cl.: G01N 33/50

(54) **Methods for identification of proteinaceous substances capable of inducing cellular reactions**

(71) Applicant: Universiteit van Amsterdam, 1018 TV Amsterdam (NL)
(72) Inventor: The designation of the inventor has not yet been filed
(74) Representative: Ottevangers, Sietse Ulbe

(57) **Abstract**

In one aspect the invention provides a method for identifying proteinaceous substances capable of inducing a reaction in a first cell upon expression and secretion by a second cell, comprising providing said second cell with a nucleic acid capable of expressing at least a functional part of a protein, co-cultivating said second cell with a first cell, while allowing for expression of said at least functional part of said protein; detecting the possibly induced reaction of said first cell and analysing the nucleic acid said second cell has been provided with.

## Description

The present invention relates to the field of molecular biology and cell biology and the application of these fields to the field of medicine. In particular the invention relates to methods of identifying proteinaceous substances which induce certain reactions in tissues exposed to these substances. More in particular it relates to such substances as produced by a group of cells, to induce a reaction, such as differentiation into a certain direction, in another group of cells adjacent to said first group of cells. Such processes are well known in nature, e.g. during embryonic development. A good example of such a process is liver development.
During early liver development several well defined stages in organogenesis can be distinguished (Zaret 1998, and others). First, pre-hepatic endoderm located in the foregut is specified towards the hepatic lineage. This event is the result of a tissue interaction between the pre-hepatic endoderm and adjacent pre-cardiac mesoderm, which will eventually give rise to the heart. The developmental relation of heart and liver was demonstrated very elegantly by LeDouarin about 30 years ago via *experimental* studies. In these experiments explants of chick embryos were cultured and cell differentiation was monitored. The outcome was that no liver tissue was found without heart cells in the vicinity showing that liver differentiation in the explants depended on the presence of cardiac tissue (Le Douarin, 1975). Later others showed the same result for mouse development. The inducing potential of mouse cardiac mesoderm was shown to be high until embryonic day 10 (Houssaint, 1980), which corresponds to embryonic day 12 in rat. Second, the newly specified hepatocytes start proliferating within the epithelial endodermal layer resulting in a regional thickening called the liver primordium. Third, hepatic cells from the liver primordium start invading the mesenchyme of the surrounding *septum transversum* in a cord-like fashion. Hereafter, the early hepatocytes position around sinusoids thus forming the liver organ.

Over the last decade, progress has been made in identifying single molecules that regulate the different stages of early liver growth utilizing molecular biological methods. In many cases likely candidates were selected based on their temporal en spatial expression pattern and studied further utilizing transgenic knock-out animals. This led to the identification of transcription factors like HNF3β, c-jun and relA and transmembrane receptors like c-met and β1-integrin (reviewed in Zaret, 1998).

In 1991 Horst *et al.* published an article in Nucleic Acids Research entitled: "A method for cDNA cloning in COS cells irrespective of subcellular site of expression". This article describes a modification of the expression cloning published by Seed *et al.* in 1987. Seed developed a method (known as 'panning') for the expression cloning of known *surface* antigens recognized by *antibodies.* Horst *et al.* made a modification allowing expression cloning of cDNAs irrespective of subcellular site of expression.

The present invention now provides methods and means to identify and/or characterize novel factors, in particular proteinaceous factors that are capable of inducing a reaction, such as (de)differentiation in certain cells. One of the objects of the invention is to identify so-called "hepatogenic factors", i.e. molecules that enhance or facilitate hepatocyte differentiation. To achieve this and other objects of the invention a novel method was developed which is not restricted to testing single candidates but provides the possibility to screen entire cDNA libraries comprehensively.

Thus the invention provides a method for identifying proteinaceous substances capable of inducing a reaction in a first cell upon expression and secretion by a second cell, providing said second cell with a nucleic acid capable of expressing at least a functional part of a protein, cocultivating said second cell with said first cell, while allowing for expression of said at least functional part of said protein, detecting the possibly induced reaction of said first cell and analysing the nucleic acid said second cell has been provided with.
As already stated Horst *et al.* published an article which describes a modification of the expression cloning published by Seed *et al.* in 1987. This method, however, still required (monoclonal) antibodies for detection of antigens encoded by the cDNA of interest. Thus only cDNAs encoding *known*, characterized proteins could be isolated.
Our method, as the others mentioned above, also includes the transfection of a cDNA library into COS cells for expression of encoded proteins. After that there is divergence. Following protein expression (and simultaneous co-cultivation with de-differentiated hepatocytes) cells are screened for the occurrence/induction of a biological event (hepatocyte re-differentiation), detectable by screening for expression of carbamoyl phosphate synthethase (CPS). The cDNAs of interest are not present in the positive cells (as above) but in cells adjacent to those. This allows the detection of novel and multiple different cDNA clones encoding/inducing a known biological funtion.
In addition to this, our method also involves an important modification with respect to plasmid recovery from screened cells. Both Seed and Horst utilize the Hirt extraction procedure for cDNA recovery after cell isolation and lysis. This method separates plasmid DNA from genomic DNA but involves time consuming centrifugation steps and expensive equipment. Since plasmid DNA could be recovered only at very low quantities utilizing the Hirt extaction, we tried a quicker and easier *total* DNA (plasmid and genomic) isolation procedure following cell isolation and lysis. Comparison of our method to the Hirt procedure revealed that high quality DNA was obtained in higer quantities. The presence of the genomic DNA did not interfere in subsequent bacterial transformation experiments.
Thus, according to the methods of the invention the detection of a substance capable of inducing a certain reaction is achieved by exposing the cells in which said reaction is supposed to occur to said substance by cocultivation of said cell that is supposed to react with a cell producing the substance that may induce said reaction, thereby closely mimicking nature. Because very many genes can be provided through cDNA expression libraries, it has now become feasible to do large scale screening for substances which do induce the expected reaction. Since the proteinaceous substance is found through expression of a gene provided to a cell, it will typically be a product directly or indirectly encoded by said gene. It may be a (poly)peptide, it may comprise glycosylation or other post-translational modifications, it may also be a complex of a polypeptide (protein) with another substance (such as RNA or carbohydrates). Cocultivation of the two celltypes, herein called producer cells and inducible cells means that the expression products of the producer cells should at least be capable of reaching the inducible cells in order to be able to induce a reaction. Of course an easy way to ensure this requirement is growing the two celltypes in one container. The methods are preferably applied in order to screen for substances that are capable of inducing dedifferentiation or differentiation (most preferred) of certain cells. They can also be used to identify substances that maintain cells in a certain state of differentiation or dedifferentiation, which would normally not be a stable steady state.
A much preferred embodiment is to screen for substances capable of changing the differentiation state of hepatocytes or cells derived therefrom or cells destined to become hepatocytes. In this way substances can be identified that can e.g. maintain hepatocytes in a differentiated state, which enables their improved application in e.g.
bioartificial livers. The present invention also provides substances identified by methods according to the invention. Thus the invention provides a proteinaceous substance capable of inducing a reaction in a first cell upon expression and secretion by a second cell, obtainable by a method according to the invention, in particular a proteinaceous substance of inducing differentiation to and/or maintaining differentiation of hepatocytes. One group of such substances is S3a or a functional analogue, homologue, derivative and/or functional fragment thereof.
S3a is a 29.8 Kd protein containing 263 amino acids (excluding the initial methionine which is removed after translation) [Kruppa et al, 1992, Zarbl et al, 1992]. S3b is identical to S3a except that it lacks the carboxy-terminal 12 residues [Wool et al, 1996]. It is not known whether S3b is an alternatively processed product of the same gene that produces S3a or is encoded by a separate gene [Wool et al, 1996]. Ribosomal protein S3a is located at the protuberance of the 40S ribosomal subunit (= small subunit) at the interface with the 60S (=large) subunit [Bielka et al, 1990, Ogata et al, 1986], and forms part of the binding site for the initiation factors eIF-2, eIF-3 and contacts both the initiator met-tRNA and mRNA (summarized in [Wool et al, 1996, Ogata et al, 1986]. S3a is therefore ideally positioned to play a role in the regulation of the initiation of protein synthesis.
The S3a gene consists of 6 exons and 5 introns [Seifart et al, 1996], lacks canonical TATA and CCAAT boxes, but instead, has a C+G-rich promoter [Seifart et al, 1996, Rebane et al, 1998]. Introns 3 and 4 contain open reading frames for the small nuclear (sno) RNA U73 [Rebane et al, 1998]. The human gene is localised on chromosome 4q.31.2-3 [Seifart et al, 1998, Rebane et al, 1998], but ≥6 processed pseudogenes exist [Kruppa et al, 1992, Wool et al, 1996, Seifart et al, 1996]. Few complete cDNAs have been isolated, but mRNAs of approx. 900 [Kruppa et al, 1992 Wool et al,1996, Moley et al, 1997 Starkey et al, 1995] and 1,300 nucleotides [Moley et al, 1997 , Szpirer et al, 1997] have been shown. The mRNA has an optimal ATG environment (ACC**AT**GG) and a consensus AAAUAAA polyadenylation signal 23 nucleotides upstream of the polyA⁺ addition site [Zarbl et al, 1992, Wool et al, 1996]. The protein is very conserved, with 99% conservation between the human, mouse and rat sequences [Zarbl et al, 1992, Seifart et al, 1996, Rebane et al, 1998], and 94% between the human and cat sequence [Levy et al, 1996]. The protein sequence is ≥60% similar to the homologous proteins in insects (Drosophila) where the protein functions in oogenesis [Kafatos et al, 1997], yeast where it functions in the transport of proteins into the mitochondria [Zarbl et al, 1992, Szpirer et al, 1997], and plants, where it is upregulated during the S-(DNA synthesis) phase of the cell cycle [Szpirer et al, 1997].

The eukaryotic ribosome contains approx. 85 proteins and 4 RNAs [Wool et al, 1979]. The coordinate, stoichiometric production of the ribosomal proteins and the rRNAs was considered crucial for proper ribosome assembly and function [Mager et al, 1988]. Nevertheless, overexpression of one or a few of the ribosomal proteins is not rare, which perhaps corresponds with the now widely accepted view that many ribosomal proteins seem to have a second function outside the ribosome [Wool et al, 1996]. For this reason, it is hypothesised that ribosomal proteins were co-opted from among a set of pre-existent proteins with other functions [Wool et al., 1996]. Thus, S3a was rediscovered several times in functional assays. S3a was identified as the v-fos-transformation effector gene (*fte-1*) by isolating revertants of v-fos (an oncogene) transformed rat-1 cells cotransfected with a human cDNA expression library [Zarbl et al, 1992, Szpirer et al, 1997]. S3a was cloned as *TU-11* after subtracting a tumor-necrosis factor α (TNFα) induced cDNA library of murine fibroblasts (CH3A cells) from a control library of the same cells [Szpirer et al, 1997, Boss et al, 1992], and as the abundantly present *nbl* mRNA in Namalwa Burkitt lymphoma [Naora et al, 1995, Naora et al, 1996]. The extra-ribosomal function of S3a has not yet been clearly defined. High levels have been measured in the human promyelocytic leukemia cell line HL60 [Naora et al, 1996], in many lymphomas [Starkey et al, 1995, Naora et al, 1995], in transformed rat-1 fibroblasts [Zarbl et al, 1992], in lymph node metastases of thyroid cancer, but not in the primary tumor [Moley et al, 1997], in (feline) fetal thymus and liver [Starkey et al, 1995], and possibly in regenerating liver [Leader et al, 1980, Sells et al, 1975]. On the other hand, very low levels are found in e.g. HepG2 hepatoblastoma cells [Naora et al, 1996], and in NIH3T3 [Naora et al, 1998] and C3HA fibroblasts [Boss et al, 1992]. Upregulation of S3a expression has been associated with cell transformation [Zarbl et al, 1992]. S3a expression is also upregulated by the cytokine TNFα [Boss et al, 1992]. The group of Noara [Naora et al, 1995, Naora et al, 1996, Naora et al, 1998 ] hypothesises that downregulation of S3a expression, from a constitutively or induced high level of expression, leads to apoptosis. A physiological example of this mechanism is seen during glucocorticoid-induced apoptosis of lymphocytes in the thymus [Noara et al, 1995].
We have now found that upon overexpression of S3a/Fos-effector protein (S3a/FEP) in (fibroblast-like) cell lines, co-cultured de-differentiated hepatocytes regain their differentiated phenotype (in the presence of hormones). Based upon this observation, S3a itself or the proteins it induces the host cell to produce to modify can be used to retain differentiated functions in primary hepatocytes in culture. One approach involves the production of a feeder cell line, which expresses S3a constitutively, and which can be used in conjunction with primary hepatocyte cultures to preserve their original degree of phenotypic differentiation in culture.
A second approach involves identification of target proteins of S3a/FEP that can be used, after purification, as additives in primary hepatocyte cultures to preserve their original degree of phenotypic differentiation. Our experiments have shown that such proteins engage in cell-cell contact. These experiments show that these proteins are either transmembrane proteins or extracellular matrix proteins, or are translocated across the cell membrane and localized to adjacent cells. Such purified proteins can be used to produce a matrix on which primary hepatocytes can be cultured, or used as an additive in such primary cultures.
A major commercial application of primary hepatocyte cultures is in a device for temporary extracorporal liver support ("bioartificial liver" or BAL) (Flendrig, 1997). For this bioartificial liver to be effective it is essential that the hepatocytes used are in a functionally differentiated state. The only way to achieve this at the moment is to refresh the hepatocytes in the apparatus every few days, which is an expensive and time-consuming approach. The availability of an agent keeping primary hepatocytes differentiated in culture avoids this step and is a major advance in the development of the bioartificial liver.
The invention thus also provides a method for keeping or bringing a group of hepatocytes in culture in an essentially differentiated state comprising contacting said hepatocytes with a proteinaceous substance according to the invention, typically wherein said group of hepatocytes is part of a bioartificial liver.
The invention further provides a bioartificial liver comprising a group of hepatocytes and a means of bringing said hepatocytes in contact with a proteinaceous substance according to the invention.

### Experimental.

### 1. Generation of a collection of gene-products containing the hepatogenic factors.

To this end, a uni-directional cDNA expression library (10⁷ independent clones) was prepared from the inducing tissue, that is from cardiac mesoderm of embryonic day 12 rats. Qualitation of the library was performed via transfection of a sample to COS-1 cells, for transient expression. Immunohistochemical staining for β-myosin heavy chain (β-MHC; 6000 basepairs), normally expressed in the heart ventricle, showed that also very large clones are represented in the cDNA library, and that these large clones are translated correctly by COS -1 cells.

### Methods employed.

### cDNA library, preparation

A *representative* cDNA expression library (> 5 x 10⁵ independent clones) is prepared from the inductive tissue. It is of importance that the clones are essentially full-length and essentially uni-directionally cloned since *biological activity* of the encoded proteins will be tested in a functional assay. This involves the detection of molecular markers for the biological activity in question via immunohistochemistry. (In our case, onset of hepatocyte formation / differentiation marked by expression of CPS). To avoid false positives one can ensure that this marker is absent in the cDNA expression library to be screened.

### cDNA library, expression

To study the biological activity of the proteins encoded by the cloned cDNAs, an *in vitro* protein expression system is used. We cloned the library into expression vector pcDNA3 and used COS-1 cells for transient expression of the proteins. The vector contains the cytomegalovirus promoter / enhancer (CMV) to drive protein expression in the COS-1 cells. In addition, there is a SV40 origin of replication present on pcDNA3. In cells expressing large T antigen (like COS-1) plasmid replication is stimulated. This increases the concentration of the protein under study and, in addition, facilitates plasmid recovery from the COS-1 cells.

### 2. Identification of hepatogenic factors in the library via an in vitro assay.

In order to identify the constructs in the cDNA library encoding a hepatogenic factor a functional assay was developed. This assay involves the co-cultivation of two different cell types:
On the one hand we use de-differentiated hepatocytes which are obtained via culturing primary embryonic day 15 rat hepatocytes for 7 days. After de-differentiation they no longer express hepatocyte-specific genes and their morphology has become fibroblast-like (Houssaint, 1976). This was confirmed by staining for carbamoyl phosphate synthetase (CPS), the first enzyme of the ornithine cycle, which is used as the marker enzyme in the assay. From experimental studies it is known that the de-differentiation process can be reversed by adding natural inducing tissue to the cells (Houssaint, 1976).
On the other hand we have COS-1 cells (monkey kidney cells) that are transfected with the cDNA expression library. These cells express the proteins encoded by the cDNA constructs, as was shown by the β-MHC experiment mentioned before. To identify transfected cells synthesizing an inducing molecule, both cell-types are cultured together under stimulation with hormones (cAMP and dexamethasone) for 48 hours. COS-1 cells producing a hepatogenic factor will act as inducing tissue in the co-cultivation, and induce re-differentiation in nearby hepatocytes. This is studied by immunohistochemical staining for induction marker CPS. The cells (areas) surrounding CPS positive cells are then harvested and plasmid DNA is retrieved, resulting in a cDNA library enriched for hepatogenic factors. After several rounds utilizing this protocol, individual clones are isolated with desired biological function, e.g. involvement in hepatocyte differentiation.

### Hepatocyte de-differentiation

De-differentiated hepatocytes were used as the responder tissue in the co-cultivation assay. These cells no longer express hepatocyte-specific genes and have a fibroblast-like appearance. The time of culturing for de-differentiation is of importance. If time is kept too short the cells appear fibroblast-like but still express hepatocyte-specific genes, resulting in false positives. If time is taken too long de-differentiation is essentially irreversible.

Other important factors in de-differentiation are cell density (high density delays de-differentiation) and cell passaging (passaging promotes de-differentiation). A well known problem, occuring when embryonic hepatocytes are passaged several times, is inability of the cells to attach to the culture dish leading to selective loss of (de-differentiating) hepatocytes. This was circumvented by using culture medium conditioned by NIH/3T3 cells, which contains unknown factors facilitating cell attachment.

### DNA recovery

To isolate cDNA clones of interest, positive responder cells and surrounding COS-1 cells are harvested and cDNA constructs are isolated. The method of choice for both marker detection and DNA isolation is important.

Analysis of marker expression, when carried out via peroxidase / immuno-histochemistry, should generally be performed utilizing 3-amino-9-ethyl-carbazole (AEC) as the substrate and not 3,3'-diamino-benzidine (DAB) in the final detection step because DAB prevents the recovery of plasmid DNA, most likely by trapping.

Isolation of plasmid DNA from transfected cells is optimal employing a protocol designed for isolation of *total* DNA. The Hirt-isolation procedure, designed for the differential isolation of exogenic and genomic DNA, gave a lesser result and is also more laborious.

### RESULTS

In the co-culture clusters of CPS-negative cells were present, surrounded by cells clearly positive for CPS. This strongly suggested that the negatively stained cells in the center are COS-1 cells, producing the inducing protein, while the surrounding CPS-positive cells are re-differentiated hepatocytes. Isolation of plasmid DNA from the cells surrounding positives, and subsequent transfection to bacteria for amplification, resulted in a cDNA library enriched for the inducing factor. After two rounds of selection and amplification, the initial cDNA library containing 10⁷ independent clones was diminished to 470 plasmids. Further analysis of this group via batch-transfections (50 plasmids) and finally mono-transfections resulted in the isolation of a single construct capable of inducing CPS-expression in the de-differentiated hepatocytes. This cDNA codes for ribosomal protein S3a. The result obtained with this single construct is reproducible and not cell-line specific since re-differentiation was observed with COS-1 cells as well as with NIH/3T3 cells. CPS expression was not observed when primary un-differentiated endoderm was used (pre-hepatic area, isolated from 7 somite rat embryos), in stead of de-differentiated hepatocytes. A positive result was also not obtained when conditioned medium taken from a COS-1 culture transfected with S3a was used, indicating that re-differentiation requires cell-cell contacts and is not triggered by a soluble factor. Statistical analysis, performed on the results obtained with experimental and control cultures, showed that this outcome is specific and not the result of a random spontaneous hepatocyte re-differentiation event.

Ribosomal protein S3a itself is known under different names: v-fos-transformation effector protein (Lecomte et al. 1997), Fte-1 or TU-11. Its mRNA consists of 880 nucleotides and, interestingly, encodes the antisense of bone morphogenetic protein 4 (BMP-4), a transcription factor known to be involved in cardiac muscle development. The S3a protein is well conserved during evolution and is localized in the cytoplasm close to the nucleus in association with the 40S ribosomal subunit. In addition to the classic role in protein synthesis, S3a has also other, recently discovered, functions: It is suspected to be involved in cell cycling, since S3a accumulates during cell cycle S-phase (DNA synthesis) (Kho, 1996), S3a is also connected to tumorigenesis: it is upregulated 5-fold in v-fos-transformed rat fibroblasts whereas inactivation of S3a reverts these cells to normal (Kho, 1992). Furthermore, S3a is also upregulated in tumor cells in vivo (Lecomte et al. 1997) whereas inhibition of S3a expression induces apoptosis (Naora, 1998). So our results and the data available from literature taken together point towards a differentiation-promoting role for S3a in the growth phase of liver development. We have also observed high levels of S3a at locations of rapid growth in tissues like the central nervous tissue, lung and intestine, indicating that the differentiation-promoting activity of S3a is not confined to the liver.

### REFERENCES

Boss, J. M., Salvo, R., Kucera, G. & Gordon, H. M. (1992) Tumor necrosis factor induces genes involved in inflammation, cellular and tissue repair, and metabolism in murine fibrolasts. *J.Immunol. 148*, 4021-4027.

Bielka, H., Noll, F., Kargel, H. J., Stahl, J. & Lutsch, G. (1990) Immunoelectron microscopic studies on the location of ribosomal proteins on the surface of the 40S ribosomal subunit from rat liver. *Eur.J.Cell Biol. 51,* 140-150.

Flendrig LM, Soe JW, Jörning GGA, Steenbeek A, Karlsen OT, Bovée WMMJ, Ladiges NCJJ, Velde AA, Chamuleau RAFM: *In vitro* evaluation of a novel bioreactor based on an integral oxygenator and a spirally wound nonwoven polyester matrix for hepatocyte culture as small aggregates. *J of Hepatology* 1997, 26: 1379-1392.

Horst, E., Wijngaard, P.L.J., Metzelaar, M., Bast, E.J.E.G. & Clevers, H.C. (1991) A method for cDNA cloning in COS cells irrespective of subcellular site of expression. *Nucl Acids Res 19,* 4556.

Houssaint E: Reversibilite de la dédifférenciation observée dans les cultures cellulaires de foie embryonnaire d'oiseau. *J Embryol Exp Morph* 1976, 35: 227-240.

Houssaint E: Differentiation of the mouse hepatic primordium. I. An analysis of tissue interactions in hepatocyte differentiation. *Cell Diff* 1980, 9: 269-279.

Kho CJ, Zarbl H: Fte-1, a v-*fos* transformation effector gene, encodes the mammalian homologue of a yeast gene involved in protein import into mitochondria. *Proc Natl Acad Sci USA* 1992, 89: 2200-2204.

Kafatos, F. C., Barajas, V., Bolshakov, V. N., Reynaud, E. & Zurita, M. (1997) Antisense suppression of the putative ribosomal protein S3a gene disrupts ovarian development in Drosophila melanogaster. *Mol.Gen.Genet. 256,* 462-467.

Kho CJ, Wang Y, Zarbl H: Effect of decreased fte-1 gene expression on protein synthesis, cell growth, and transformation. *Cell Growth and Diff* 1996, 7: 1157-1166.

Kruppa, J., Stahl, J., Pooga, M., Hoth, S., Rebane, A. & Metspalu, A. (1992) Human ribosomal protein S3a: cloning of the cDNA and primary structure of the protein. *Gene 119,* 313-316.

Leader, D. P. (1980) Phosphorylated and other modified forms of eukaryotic ribosomal protein S3 analysed by two-dimensional gel electrophoresis. *Biochem.J. 189,* 241-245.

Lecomte F, Szpirer J, Szpirer C: The S3a ribosomal protein gene is identical to the *Fte-1* (v-*fos* transformation effector) gene and the TNF-α-induced *TU-11* gene, and its transcript level is altered in transformed and tumor cells. *Gene* 1997, 186: 271-277.

Le Douarin NM: An experimental analysis of liver development. *Med Biol* 1975, 53: 427-455.

Levy, L. S., Prabhu, S., Menon, R. P. & Starkey, C. R. (1996) Primary Sequence and Evolutionary Conservation of Ribosomal Protein Genes from the Domestic Cat.
*Biochem.Biophys.Res.Commun. 220,* 648-652.
Mager, W. H. (1988) Control of ribosomal protein gene expression. *Biochim.Biophys.Acta 949,* 1-15.

Moley, J. F., Wells, S. A., Pichmayr, M. D., Scheumann, G. F. W., Goodfellow, P. J. & Musholt, T. J. (1997) Differential Display in Primary and Metastatic Medullary Thyroid Carcinoma. *J.Surg.Res. 69,* 94-100.

Naora, H., Naora, H., Nishida, T., Shindo, Y. & Adachi, M. (1995) Association of nbl gene expression and glucocorticoid-induced apoptosis in mouse thymus in vivo. *Immunology 85,* 63-68.

Naora, H., Adachi, M., Shindo, Y., Nishida, T. & Naora, H. (1996) Constitutively Enhanced nbl Expression is Associated with the Induction of Internucleosomal DNA Cleavage by Actinomycin D. *Biochem.Biophys.Res.Commun. 224,* 258-264.

Naora, H., Adachi, M., Takai, I. & Naora, H. (1998) Altered Cellular Responses by Varying Expression of a Ribosomal Protein Gene: Sequential Coordination of Enhancement and Suprpression of Ribosomal Protein S3a Gene Expression Induces Apoptosis. *J Cell Biol 141,* 741-753.

Naora H, Takai I, Adachi M, Naora H: Altered cellular responses by varying expression of a ribosomal protein gene: sequential coordination of enhancement and suppression of ribosomal protein S3a gene expression induces apoptosis. *J Cell Biol* 1998, 8: 741-753.

Ogata, K., Kikuchi, M. & Uchiumi, T. (1986) Cross-linking Study on Protein Neighborhoods at the Subunit Interface of Rat Liver Ribosomes with 2-Iminothiolane. *The Journal of Biological Chemistry 261,* 9663-9667.

Rebane, A., Metspalu, A., Pata, I., Laan, M. & Tamme, R. (1998) A novel snoRNA (U73) is encoded within the introns of the human and mouse ribosomal protein S3a genes. *Gene 210,* 255-263.

Seed, B., & Aruffo, A. (1987) Molecular cloning of the CD2 antigen, the T-cell erythrocyte receptor, by a rapid immunoselection procedure. *Proc Natl Sci USA 84,* 3365-3369.

Seifart, K. H., Kalff-Suske, M., Taimor, G. & Nolte, D. (1996) The human S3a ribosomal protein: sequence, location and cell-free transcription of the functional gene. *Gene 169,* 179-185.

Sells, B. H., Grundholm, A. & Anderson, W. M. (1975) Modification of ribosomal proteins during liver regeneration. *Biochem.Biophys.Res.Commun. 62,* 669-676.

Starkey, C. R. & Levy, L. S. (1995) Identification of differentially expressed genes in T-lymphoid malignancies in an animal model system. *Int.J.Cancer 62,* 325-331.

Szpirer, C., Lecomte, F. & Szpirer, J. (1997) The S3a ribosomal protein gene is identical to the Fte-1 (v-fos transformation effector) gene and the TNF- alpha- induced TU-11 gene, and its transcript level is altered in transformed and tumor cells. *Gene 186,* 271-277.

Wool, I. G., Paz, V., Olvera, J. & Chan, Y. L. (1996) The Primary Structures of Rat Ribosomal Proteins S3a (The V-Fos Transformation Effector) and of S3b.
*Biochem.Biophys.Res.Commun. 228,* 141-147.

Wool, I. G. (1979) The structure of eucaryotic ribosomes. *Ann.Rev.Biochem. 48,* 719-754.

Wool, I. G. (1996) Extraribosomal functions of ribosomal proteins. *Trends Biochem.Sci. 21,* 164-165.

Zarbl, H. & Kho, C. J. (1992) Fte-1, a v-fos transformation effector gene, encodes the mammalian homoloque of a yeast gene involved in protein import into mitochondria. *Proc.Natl.Acad:Sci. 89,* 2200-2204.

Zaret K: Early liver differentiation: genetic potentiation and multilevel growth control. *Curr Opin in Genet & Dev* 1998, 8: 526-531.

## Claims

1. A method for identifying proteinaceous substances capable of inducing a reaction in a first cell upon expression and secretion by a second cell, comprising providing said second cell with a nucleic acid capable of expressing at least a functional part of a protein, cocultivating said second cell with said first cell, while allowing for expression of said at least functional part of said protein, detecting the possibly induced reaction of said first cell and analysing the nucleic acid said second cell has been provided with.

2. A method according to claim 1, wherein said reaction is dedifferentiation or differentiation of said first cell or prevention of either in said first cell.

3. A method according to claim 1 or 2, wherein said first cell is a cell derived from a hepatocyte.

4. A method according to claim 3, wherein said first cell is derived from a hepatocyte by dedifferentiation.

5. A method according to claim 4, wherein said reaction is differentiation of said first cell.

6. A method according to claim 5, wherein said proteinaceous substance is capable of inducing differentiation of cells derived from hepatocytes by dedifferentiation.

7. A proteinaceous substance capable of inducing a reaction in a first cell upon expression and secretion by a second cell, obtainable by a method according to any one of claims 1-6.

8. A proteinaceous substance according to claim 7, capable of inducing differentiation to and/or maintaining differentiation of hepatocytes.

9. A proteinaceous substance according to claim 8, which is S3A or a fucntional analogue, homologue, derivative and/or functional fragment thereof for use as a medicine.

10. A method for keeping or bringing a group of hepatocytes in culture in an essentially differentiated state comprising contacting said hepatocytes with a proteinaceous substance according to any one of claims 7-9.

11. A method according to claim 10, wherein said group of hepatocytes is part of a bioartificial liver.

12. A bioartificial liver comprising a group of hepatocytes and a means of bringing said hepatocytes in contact with a proteinaceous substance according to any one of claims 7-9.
